# EUROPEAN PATENT SPECIFICATION

(11) **EP 2 612 654 B1**
(45) Date of publication and mention of the grant of the patent: **02.01.2019**
(21) Application number: 11822113.4
(22) Date of filing: 30.08.2011
(51) Int. Cl.: A61K 8/9789, A61Q 19/00, A61Q 19/08, A61K 8/64, A61K 8/14

(54) **COSMETIC COMPOSITION FOR IMPROVING SKIN ELASTICITY**
KOSMETISCHE ZUSAMMENSETZUNG ZUR VERBESSERUNG DER HAUTELASTIZITÄT
COMPOSITION COSMÉTIQUE POUR AMÉLIORER L'ÉLASTICITÉ DE LA PEAU

(30) Priority: 31.08.2010 KR 20100084506
(43) Date of publication of application: 10.07.2013
(73) Proprietor: Amorepacific Corporation, Seoul 140-777 (KR)
(72) Inventor: SON, Eui Dong, Yongin-si Gyeonggi-do 446-779 (KR); MIN, Dae Jin, Seoul 133-848 (KR); CHANG, Hui Kyoung, Yongin-si Gyeonggi-do 446-760 (KR); CHOI, Hyun Jung, Suwon-si Gyeonggi-do 443-470 (KR); CHO, Seong A, Seoul 137-769 (KR); KIM, Ji Hyun, Yongin-si Gyeonggi-do 446-905 (KR); LEE, Tae Ryong, Yongin-si Gyeonggi-do 446-729 (KR)
(74) Representative: Bugnion Genève
(86) International application number: PCT/KR2011/006405
(87) International publication number: WO 2012/030138

(56) References cited:
- WO-A1-2010/032199
- JP-A- 2006 062 991
- KR-A- 20040 059 004
- KR-A- 20100 023 372
- KR-A- 20100 023 372
- US-A1- 2010 098 773
- US-A1- 2010 215 726

## Description

### BACKGROUND

### 1. Field

The present disclosure relates to a cosmetic composition for improving skin elasticity.

### 2. Description of the Related Art

Structural changes of the epidermis, dermis, etc. result in reduced elasticity and drooping of the skin.

The thickness of the dermis decreases gradually. Total collagen content in the dermis decreases by 1% in a year after being adults and the remaining collagen fibers become gradually thicker, leading to increased crosslinking and decreased solubility, extensibility, etc.

In addition, elastin becomes thicker and more crosslinked. Besides, proliferative activity of fibroblasts in the dermis decreases whereas collagen synthesizing ability decreases and degrading ability increases.

As the regeneration of the epidermis, dermis, etc. becomes slow and the adhesion between the epidermis and the dermis becomes weak, the skin elasticity is decreased rapidly.

Although efforts have been made to improve the condition of skin elasticity and so on for women by increasing collagen or elastin, there have been few studies on the adhesion between the epidermis and the dermis.

### SUMMARY

The present disclosure is directed to providing a cosmetic composition for improving skin elasticity containing *Phyllanthus urinaria* extract and a polymersome in which an anti-aging peptide is stabilized as active ingredients and efficacious in improving skin wrinkles and restoring skin elasticity.

The present invention is directed to a cosmetic use of a composition for improving skin elasticity comprising *Phyllanthus urinaria* extract and a polymersome in which an anti-aging peptide is captured as active ingredients,
wherein the anti-aging peptide is palmitoyl tripeptide-5 or diaminopropionoyl tripeptide 33, and
wherein the polymersome is synthesized from various amphiphilic polymers having both hydrophobic and hydrophilic blocks.

The cosmetic composition of the present disclosure is efficacious in improving skin wrinkles, restoring skin elasticity and increasing skin water content, and thus is effective for improving skin elasticity.

### BRIEF DESCRIPTION OF THE DRAWINGS

FIG. 1 shows change in the level of perlecan gene in normal human fibroblasts when treated with *Phyllanthus urinaria* extract and ABcell™;
FIG. 2 shows change in the level of perlecan protein in normal human fibroblasts when treated with *Phyllanthus urinaria* extract;
FIG. 3 shows change in perlecan protein isolated from normal human fibroblasts of a adult in his 20s as fluorescence intensity after immunofluorescence staining;
FIG. 4 shows change in perlecan protein isolated from normal human fibroblasts of a adult in his 40s as fluorescence intensity after immunofluorescence staining;
FIG. 5 shows change in perlecan protein isolated from normal human fibroblasts of a adult in his 40s as fluorescence intensity after immunofluorescence staining when treated with ABcell™;
FIG. 6 shows a result of a clinical trial on skin wrinkles carried out by DERMAPRO Co., LTD., an independent clinical trial institute, for cosmetic formulations containing *Phyllanthus urinaria* extract and ABcell; and
FIG. 7 shows a result of a clinical trial on the improvement of skin elasticity carried out by Dermapro for cosmetic formulations containing *Phyllanthus urinaria* extract and ABcell.

### DETAILED DESCRIPTION

Exemplary embodiments now will be described more fully hereinafter with reference to the accompanying drawings, in which exemplary embodiments are shown.

The present disclosure provides a cosmetic use of a composition for improving skin elasticity comprising *Phyllanthus urinaria* extract and a polymersome in which an anti-aging peptide is captured as active ingredients,
wherein the anti-aging peptide is palmitoyl tripeptide-5 or diaminopropionoyl tripeptide 33, and
wherein the polymersome is synthesized from various amphiphilic polymers having both hydrophobic and hydrophilic blocks.

*Phyllanthus urinaria* is also called chamberbitter, gripeweed and usually grows in fields or grasslands. *Phyllanthus urinaria* is known to be effective in treating enteritis, dysentery, edema caused by infectious hepatitis and nephritis, urinary tract infection, brightening eyes, infantile malnutrition, acute inflammation of eyes or corneal opacity, mouth ulcer, smallpox and occurrence of unknown furunculus in body and to provide a skin-whitening effect when included in cosmetics. Korean Patent Application Publication No. 2004-59004 discloses a skin whitening composition with inhibitory activity of melanogenesis, which contains one or more plant extracts selected from a group consisting of *Phyllanthus urinaria* extract, *Alocasia cucullata* extract and a mixture thereof as an active ingredient. Japanese Patent Publication No. H08-12566 discloses an inhibitor of tyrosinase activity containing one or more selected from a group consisting of *Phyllanthus niruri* L. extract, matico (*Piper elongatum* Vahl. & *Piper ungustifolium*) extract and *Urtica urens* extract.

KR-A-2010/0023372 is directed to cosmetic compositions containing Phyllanthus urinaria extract as an active ingredient for anti-aging, anti-oxidation, anti-wrinckle effects. This document does not disclose the presence of a polymersome comprising an anti-aging peptide.

US-A-2010/215726 discloses cosmetic skin care compositions including one or a combination of natural and/or synthetic protein and/or peptide ingredients that function in a synergistic manner to rejuvenate and condition the skin as well as to reduce the appearance of wrinkles. More particularly the cosmetic composition for topical application to the skin, comprises as active agents: acetyl hexpeptide; palmitoyl tripeptide-3; palmitoyl oligopeptide; palmitoyl tetrapeptide-7 ; dipeptide diaminobutyroyl benzylamide diacatate; acetyl glutamyl heptapeptide-1; and a cosmetically acceptable vehicle.

WO-A-2010/032199 aims to a provide a new type of polymersomes allowing effective control of the release of the asset it contains in response to an external stimulus that is readily applicable to a topical manner and/or in vivo. These polymersomes are used for encapsulating, transporting and releasing in a controlled manner one or more hydrophilic active agents.

The anti-aging peptide refers to a peptide exhibiting an anti-aging effect and are, for example, palmitoyl tripeptide-5 or diaminopropionoyl tripeptide 33. The polymersome in which an anti-aging peptide is stabilized may be ABcell™ (Amorepacific).

A polymersome is an effective vesicle-type nanostructure synthesized from various amphiphilic polymers having both hydrophobic and hydrophilic blocks. In an aqueous solution, the amphiphilic polymers form aggregates according to the property of the hydrophilic blocks tending to aggregate together to decrease the free energy of the system. Since the hydrophilic blocks are uniformly dissolved in the aqueous solution, the polymersome may maintain a thermodynamically stable structure in the aqueous solution. The polymersome exhibits superior ability of penetrating into the skin and capturing active ingredients and is capable of maintaining the structure for a long period of time upon administration into the body because it is remarkably stable in aqueous solutions.

The composition of the present disclosure may increase expression of the perlecan gene and increase production of the perlecan protein. Perlecan is a proteoglycan existing in the epidermis and the dermis and has been found, with various growth factors attached thereto, to affect proliferation, differentiation and adhesion of epidermal cells. The composition of the present disclosure increases expression of the perlecan gene, thereby maintaining the skin structure by promoting regeneration of the epidermis and the dermis and the improvement of adhesion between the epidermis and the dermis. Accordingly, it may improve and restore skin elasticity.

Further, the composition restores production of the perlecan protein decreased by UV.

The composition of the present disclosure improves skin wrinkles, restores skin elasticity and increases skin water content.

### [Example 1] Experiment for increase of perlecan gene (isolation of RNA and RT-PCR)

Fibroblasts obtained from a newborn infant were seeded onto a 60-mm cell culture dish using DMEM containing 10% serum at a density of 1.25×10⁶ cells/dish and cultured at 37 °C in a 5% CO₂ incubator to about 80% confluency. After starvation for 24 hours, the cells were treated with *Phyllanthus urinaria* extract and ABcell™ at various concentrations, which had been washed twice with PBS, and cultured for 2 days. After removing the medium, RNA was isolated according to the Invitrogen's RNA separation method by adding 1 mL of Trizol (Invitrogen). After quantifying RNA at 260 nm using a UV detector (Hewlett Packard), reverse transcription-polymerase chain reaction (RT-PCR) was carried out. For genetic analysis of each sample, correction was made using the complementary 36B4 gene. The primer sequences of perlecan are as follows.
Sense: 5'- ctgagtgatgcaggcaccta-3' (SEQ ID NO: 1)
Antisense: 5'- ctctctgggctcacttggac-3'(SEQ ID NO: 2)

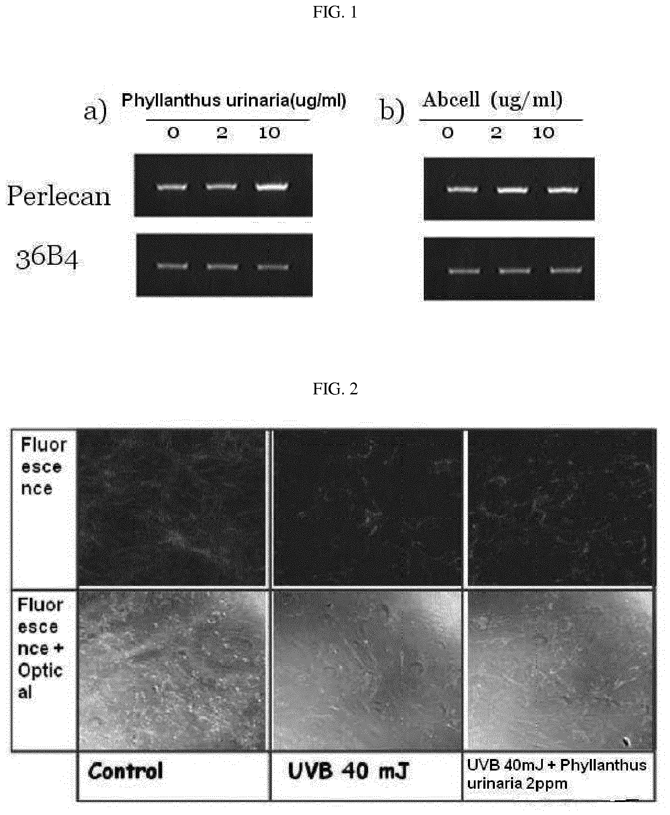

As seen from FIG. 1, *Phyllanthus urinaria* extract and ABcell resulted in increased level of perlecan in the fibroblasts.

### [Example 2] Change in perlecan using immunofluorescence staining

Normal human fibroblasts were seeded onto a 60-mm cell culture dish using DMEM containing 10% serum at a density of 1.25×10⁶ cells/dish and cultured at 37 °C in a 5% CO₂ incubator to about 80% confluency. After starvation for 24 hours, the cells were washed twice with PBS and cultured for 2 days while irradiating UV B and treating with *Phyllanthus urinaria* extract. Then, increasing situation of the perlecan protein in cell status was investigated.

Adult human dermal fibroblasts (HDFa) purchased from Cascade Biologics (USA) were cultured using M106 medium (Cascade Biologics, USA) at 37 °C in a 5% CO₂ incubator.

After spotting the cells onto a slide glass for immunofluorescence staining and treating with a substance for 48 hours, immunofluorescence staining was carried out. Details about the immunofluorescence staining are as follows. The cells were washed twice with DPBS and then fixed by treating with 3.5% paraformaldehyde for 10 minutes. The fixed cells were washed 3 times with DPBS, for 10 minutes each, and treated with 0.1% Triton X-100 for 5 minutes for permeation into the cells. After washing with PBS for 10 minutes, the cells were blocked with 5% goat serum for 30 minutes. After the blocking, the cells were treated with 5% goat serum with primary antibody added. Then, incubation was performed at room temperature for 1 hour so that the primary antibody (anti-perlecan antibody, Santa Cruz Biotechnology, USA) could bind to the corresponding antibody. After removing surplus primary antibody by washing 3 times with DPBS, for 10 minutes each, the cells were treated with secondary antibody at room temperature for 30 minutes. Surplus secondary antibody was completely removed by washing 3 times with DPBS, for 10 minutes each. After dropping one drop of a mounting solution onto a slide glass, followed by covering with a cover slip, the surplus mounting solution leaking out of the cover slip was removed and the cover slip was sealed.

Then, difference in fluorescence of each test group was observed using a confocal microscope.

As a result, it was confirmed that UV B resulted in decrease of perlecan and the *Phyllanthus urinaria* extract restored the production of perlecan decreased by UV (FIG. 2).

### [Example 3] Experiment for restoration of perlecan level by ABcell™

Normal human fibroblasts (NHFs; isolated from adults in 20 and 40 years old) were seeded onto a 60-mm cell culture dish using DMEM containing 10% serum at a density of 1.25×10⁶ cells/dish and cultured at 37 °C in a 5% CO₂ incubator to about 80% confluency. The cultured cells were treated with 1% FBS medium + Cytokinol 100 ppm + 10% BASF for 48 hours and observed after perlecan staining. ABcell™ was treated at a concentration of 10 ug/mL. The procedure was similar to that of Example 4.

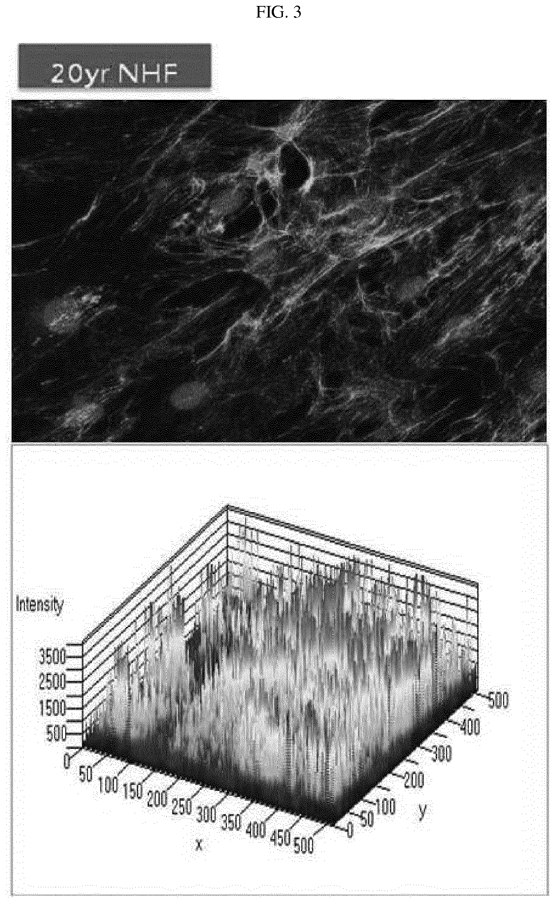

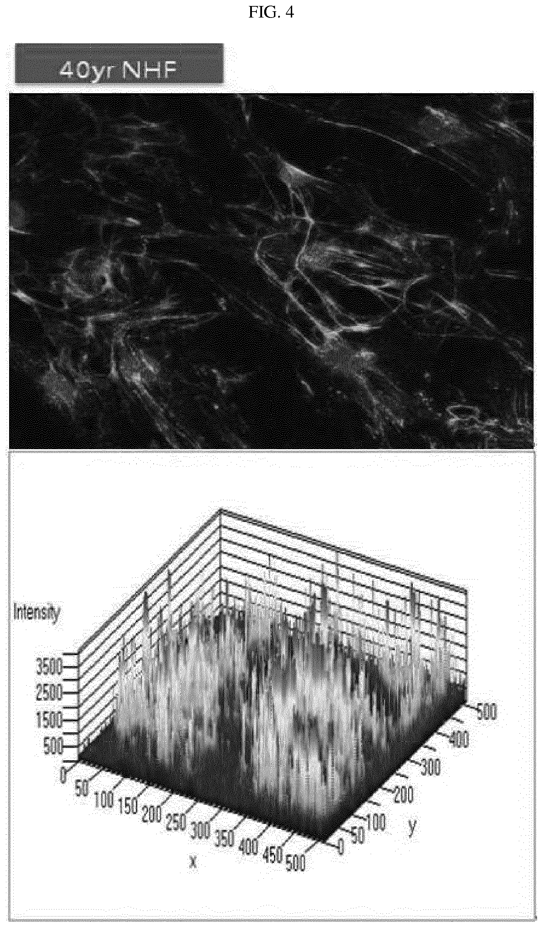

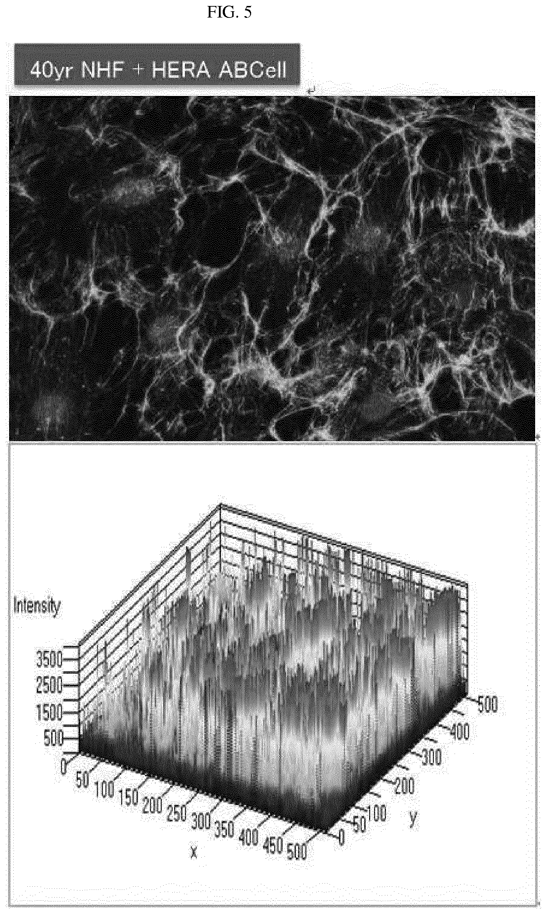

A result of measuring fluorescence intensity is shown in Table 1 and FIGS. 3-5. It was confirmed that the level of perlecan was decreased lower in the NHFs of the 40-year-old adult than in the NHFs of the 20-year-old adult (FIG. 3 and FIG. 4), and the decreased level of perlecan in the NHFs of the 40-year-old adult was restored to the level of the NHFs of the 20-year-old adult by HERA™ ABcell (FIG. 4 and FIG. 5).

**Table 1**

| | 20-yr NHF | 40-yr NHF | 40-yr NHF + ABcell |
|---|---|---|---|
| Intensity (Perlecan) | 756 | 544 | 937 |
| Relative value to 20-yr NHF | 100 | 72 | 124 |
| Relative value to 40-yr NHF | 139 | 100 | 172 |

### [Example 4] Improvement of skin wrinkles and elasticity

A clinical trial was conducted by Dermapro (Seongnam, Korea), an independent clinical trial institute, for the effect of improving skin wrinkles and elasticity of a cosmetic formulation containing *Phyllanthus urinaria* extract and ABcell. 40 women in their 30s and 40s were divided into two groups, 20 people each, and were asked to apply the formulation on the face twice a day, in the morning and evening, for 12 weeks. Then, improvement of skin wrinkles and elasticity were tested for 8 weeks using replicas according to arbitrary units (R1-R5).

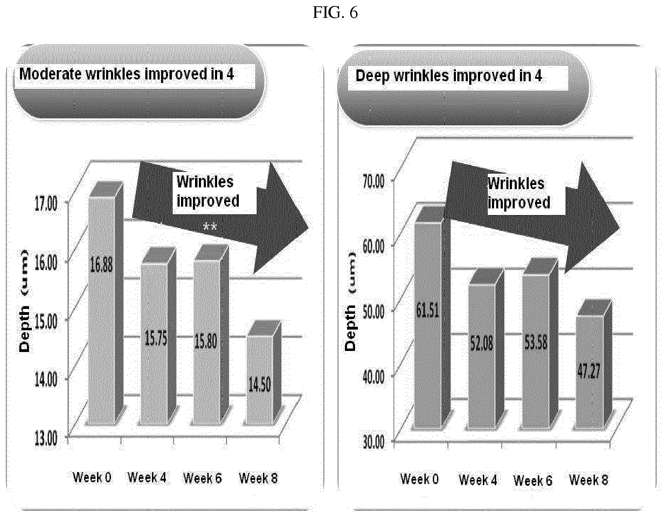

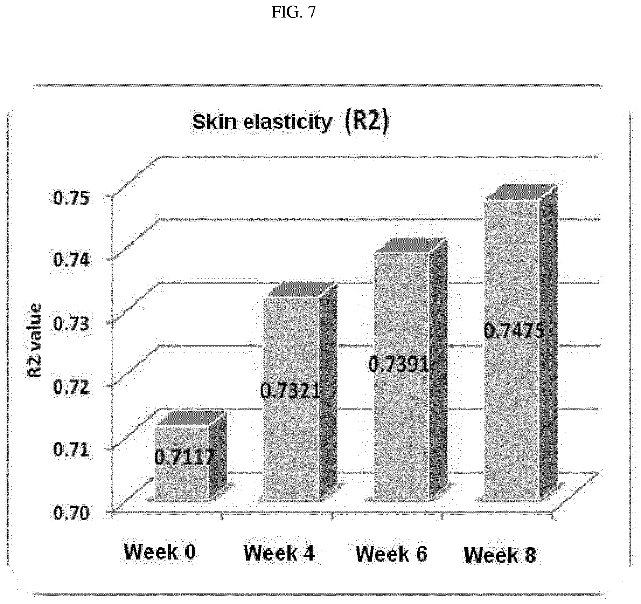

The effect of improving skin wrinkles and elasticity was observed from 4 weeks after the application of the formulation (FIG. 6 and FIG. 7).

### [Example 5] Improvement of skin water content

Skin moisturizing effect of the formulations of Comparative Example 1 and Example 1 described in Table 2 was evaluated as follows. 40 women in their 30s and 40s were divided into two groups, 20 people each, and were asked to apply the formulation on the face twice a day, in the morning and evening, for 12 weeks. Then, skin water content was measured using a corneometer(Germany). The result is given in Table 3.

**Table 2**

| Ingredients | Comparative Example 1 | Example 1 |
|---|---|---|
| Purified water | balance | balance |
| *Phyllanthus urinaria* extract | - | 0.1 |
| ABcell | - | 0.1 |
| Hydrogenated vegetable oil | 1.5 | 1.5 |
| Stearic acid | 0.6 | 0.6 |
| Glyceryl stearate | 1.0 | 1.0 |
| Stearyl alcohol | 2.5 | 2.5 |
| Polyglyceryl-10 pentastearate, behenyl alcohol & sodium stearoyl lactylate | 1.0 | 1.0 |
| Arachidyl behenyl alcohol & arachidyl glucoside | 1.0 | 1.0 |
| Cetearyl alcohol & cetearyl glucoside | 2.0 | 2.0 |
| PEG-100 stearate, glycerol oleate & propylene glycol | 1.5 | 1.5 |
| Caprylic/capric triglyceride | 11.0 | 11.0 |
| Cyclomethicone | 6.0 | 0.6 |
| Antiseptic, fragrance | adequate | adequate |
| Triethanolamine | 0.1 | 0.1 |

**Table 3**

| Test substance | Corneometer value | | |
|---|---|---|---|
| | Week 0 | Week 4 | Week 8 |
| Comparative Example 1 | 21±4 | 23±5 | 23±3 |
| Example 1 | 20±5 | 27±6 | 33±5 |

Formulation examples of the cosmetic composition and the pharmaceutical composition according to the present disclosure are described below. However, the following examples are for illustrative purposes only and not intended to limit the scope of the present disclosure.

### [Formulation Example 1] Softening lotion (skin lotion)

A softening lotion was prepared according to a commonly employed method with the composition described in Table 4.

**Table 4**

| Ingredients | Contents (wt%) |
|---|---|
| *Phyllanthus urinaria* extract | 0.1 |
| ABcell™ | 0.1 |
| Glycerin | 3.5 |
| Oleyl alcohol | 1.5 |
| Ethanol | 5.5 |
| Polysorbate 80 | 3.2 |
| Carboxyvinyl polymer | 1.0 |
| Butylene glycol | 2.0 |
| Propylene glycol | 2.0 |
| Antiseptic, fragrance | adequate |
| Purified water | balance |
| Total | 100 |

### [Formulation Example 2] Nourishing lotion (milk lotion)

A nourishing lotion was prepared according to a commonly employed method with the composition described in Table 5.

**Table 5**

| Ingredients | Contents (wt%) |
|---|---|
| *Phyllanthus urinaria* extract | 0.1 |
| ABcell™ | 0.1 |
| Glycerin | 3.0 |
| Butylene glycol | 3.0 |
| Propylene glycol | 3.0 |
| Carboxyvinyl polymer | 0.1 |
| Beeswax | 4.0 |
| Polysorbate 60 | 1.5 |
| Caprylic/capric triglyceride | 5.0 |
| Squalane | 5.0 |
| Sorbitan sesquioleate | 1.5 |
| Cetearyl alcohol | 1.0 |
| Triethanolamine | 0.2 |
| Antiseptic, fragrance | adequate |
| Purified water | balance |
| Total | 100 |

### [Formulation Example 3] Nourishing cream

A nourishing cream was prepared according to a commonly employed method with the composition described in Table 6.

**Table 6**

| Ingredients | Contents (wt%) |
|---|---|
| *Phyllanthus urinaria* extract | 0.1 |
| ABcell™ | 0.1 |
| Glycerin | 3.5 |
| Butylene glycol | 3.0 |
| Liquid paraffin | 7.0 |
| β-Glucan | 7.0 |
| Carbomer | 0.1 |
| Caprylic/capric triglyceride | 3.0 |
| Squalane | 5.0 |
| Cetearyl glucoside | 1.5 |
| Sorbitan stearate | 0.4 |
| Polysorbate 60 | 1.2 |
| Triethanolamine | 0.1 |
| Antiseptic, fragrance | adequate |
| Purified water | balance |
| Total | 100 |

### [Formulation Example 4] Massage cream

A massage cream was prepared according to a commonly employed method with the composition described in Table 7.

**Table 7**

| Ingredients | Contents (wt%) |
|---|---|
| *Phyllanthus urinaria* extract | 0.1 |
| ABcell™ | 0.1 |
| Glycerin | 8.0 |
| Butylene glycol | 3.0 |
| Liquid paraffin | 45.0 |
| β-Glucan | 7.0 |
| Carbomer | 0.1 |
| Caprylic/capric triglyceride | 3.0 |
| Beeswax | 4.0 |
| Cetearyl glucoside | 1.5 |
| Sorbitan sesquioleate | 0.9 |
| Paraffin | 1.5 |
| Antiseptic, pigment, fragrance | adequate |
| Purified water | balance |
| Total | 100 |

### [Formulation Example 5] Pack

A pack was prepared according to a commonly employed method with the composition described in Table 8.

**Table 8**

| Ingredients | Contents (wt%) |
|---|---|
| *Phyllanthus urinaria* extract | 0.1 |
| ABcell™ | 0.1 |
| Glycerin | 4.0 |
| Polyvinyl alcohol | 15.0 |
| Hyaluronic acid extract | 5.0 |
| β-Glucan | 7.0 |
| Allantoin | 0.1 |
| Nonyl phenyl ether | 0.4 |
| Polysorbate 60 | 1.2 |
| Ethanol | adequate |
| Antiseptic, fragrance | adequate |
| Purified water | balance |
| Total | 100 |

### [Formulation Example 6] Patch

A patch was prepared according to a commonly employed method with the composition described in Table 9.

**Table 9**

| Ingredients | Contents (wt%) |
|---|---|
| *Phyllanthus urinaria* extract | 0.1 |
| ABcell™ | 0.1 |
| β-1,3-Glucan | 3.0 |
| Diethylamine | 0.7 |
| Sodium sulfite | 0.1 |
| Polyoxyethylene lauryl ether (E.O = 9) | 1.0 |
| Polyhydroxyethylene cetyl stearyl ether (Cetomacrogol 1000) | 1.0 |
| Viscous paraffin oil | 2.5 |
| Caprylic/capric ester (Cetiol LC) | 2.5 |
| Polyethylene glycol 400 | 3.0 |
| Polyacrylic acid (Carbopol 934P) | 1.0 |
| Purified water | balance |
| Total | 100 |

While the exemplary embodiments have been shown and described, it will be understood by those skilled in the art that various changes in form and details may be made thereto without departing from the spirit and scope of the present disclosure as defined by the appended claims.

In addition, many modifications can be made to adapt a particular situation or material to the teachings of the present disclosure without departing from the essential scope thereof. Therefore, it is intended that the present disclosure not be limited to the particular exemplary embodiments disclosed as the best mode contemplated for carrying out the present disclosure, but that the present disclosure will include all embodiments falling within the scope of the appended claims.

## Claims

1. A cosmetic use of a composition for improving skin elasticity comprising *Phyllanthus urinaria* extract and a polymersome in which an anti-aging peptide is captured as active ingredients,
wherein the anti-aging peptide is palmitoyl tripeptide-5 or diaminopropionoyl tripeptide 33, and
wherein the polymersome is synthesized from various amphiphilic polymers having both hydrophobic and hydrophilic blocks.

2. The cosmetic use according to claim 1, wherein the composition maintains skin structure by increasing expression of the perlecan gene and thereby promoting regeneration of the epidermis and the dermis and the improvement of adhesion between the epidermis and the dermis.

3. The cosmetic use according to claim 1, wherein the composition increases production of the perlecan protein.

4. The cosmetic use according to claim 1, wherein the composition restores production of the perlecan protein decreased by UV.

5. The cosmetic use according to claim 1, wherein the composition improves skin wrinkles, restores skin elasticity and increases skin water content.

## Patentansprüche

1. Kosmetische Verwendung einer Zusammensetzung zur Verbesserung der Elastizität der Haut, umfassend einen Extrakt von *Phyllanthus urinaria* und ein Polymersom, in dem ein Anti-Aging-Peptid als aktiver Bestandteil eingefangen ist,
wobei das Anti-Aging-Peptid Palmitoyl Tripeptid-5 oder Diaminopropionoyl Tripeptid-33 ist, und
wobei das Polymersom aus verschiedenen amphiphilen Polymeren synthetisiert ist, die sowohl hydrophobe als auch hydrophile Blöcke aufweisen.

2. Kosmetische Verwendung nach Anspruch 1, wobei die Zusammensetzung die Hautstruktur durch die Erhöhung der Expression des Perlecan-Gens aufrechterhält und dadurch die Regenerierung der Epidermis und der Dermis und die Verbesserung der Haftung zwischen der Epidermis und der Dermis fördert.

3. Kosmetische Verwendung nach Anspruch 1, wobei die Zusammensetzung die Erzeugung des Perclean-Proteins erhöht.

4. Kosmetische Verwendung nach Anspruch 1, wobei die Zusammensetzung die Erzeugung des Perlecan-Proteins, verringert durch UV, wiederherstellt.

5. Kosmetische Verwendung nach Anspruch 1, wobei die Zusammensetzung Hautfalten verbessert, die Hautelastizität wiederherstellt und den Wassergehalt der Haut erhöht.

## Revendications

1. Utilisation cosmétique d'une composition pour améliorer l'élasticité de la peau comprenant un extrait de *Phyllanthus urinaria* et un polymersome dans lequel un peptide anti-âge est capturé en tant que principes actifs,
dans laquelle le peptide anti-âge est un palmitoyl tripeptide-5 ou un diaminopropionoyl tripeptide 33, et
dans laquelle le polymersome est synthétisé à partir de divers polymères amphiphiles comportant à la fois des blocs hydrophobes et hydrophiles.

2. Utilisation cosmétique selon la revendication 1, dans laquelle la composition conserve la structure de la peau en augmentant l'expression du gène du perlecan et en favorisant ainsi la régénération de l'épiderme et du derme et l'amélioration de l'adhérence entre l'épiderme et le derme.

3. Utilisation cosmétique selon la revendication 1, dans laquelle la composition augmente la production de la protéine perlecan.

4. Utilisation cosmétique selon la revendication 1, dans laquelle la composition rétablit la production de la protéine perlecan réduite par les UV.

5. Utilisation cosmétique selon la revendication 1, dans laquelle la composition améliore les rides de la peau, rétablit l'élasticité de la peau et augmente la teneur en eau de la peau.
